(19) Europäisches Patentamt
European Patent Office
Office européen des brevets

(11) **EP 2 349 405 B1**

(12) **EUROPÄISCHE PATENTSCHRIFT**

(45) Veröffentlichungstag und Bekanntmachung des Hinweises auf die Patenterteilung:
**20.11.2019 Patentblatt 2019/47**

(21) Anmeldenummer: **08802482.3**

(22) Anmeldetag: **22.09.2008**

(51) Int Cl.:
*G01F 1/36* (2006.01)     *G01F 1/38* (2006.01)
*G01F 1/40* (2006.01)     *G01F 1/50* (2006.01)
*A61M 5/168* (2006.01)

(86) Internationale Anmeldenummer:
**PCT/EP2008/007988**

(87) Internationale Veröffentlichungsnummer:
**WO 2010/031424 (25.03.2010 Gazette 2010/12)**

(54) **VORRICHTUNG ZUM BESTIMMEN ZUMINDEST EINES STRÖMUNGSPARAMETERS**

DEVICE FOR DETERMINING AT LEAST ONE FLOW PARAMETER

DISPOSITIF DE DÉTERMINATION D'AU MOINS UN PARAMÈTRE D'ÉCOULEMENT

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(43) Veröffentlichungstag der Anmeldung:
**03.08.2011 Patentblatt 2011/31**

(73) Patentinhaber: **Fraunhofer-Gesellschaft zur Förderung der angewandten Forschung e.V. 80686 München (DE)**

(72) Erfinder: **RICHTER, Martin 81677 München (DE)**

(74) Vertreter: **Stöckeler, Ferdinand Schoppe, Zimmermann, Stöckeler Zinkler, Schenk & Partner mbB Patentanwälte Radlkoferstrasse 2 81373 München (DE)**

(56) Entgegenhaltungen:
EP-A- 1 772 162     WO-A-96/03168
WO-A-2005/119181     WO-A1-03/074121
DE-A1- 4 308 313     US-A1- 2005 267 413

**Beschreibung**

Ausführungsbeispiele der vorliegenden Erfindung beziehen sich auf Vorrichtungen zum Erfassen zumindest eines Strömungsparameters und insbesondere Vorrichtungen, die zur Bestimmung einer Strömungsrate und/oder einer Verstopfung einer Flussrestriktion geeignet sind.

Ausführungsbeispiele der Erfindung eignen sich insbesondere zur Verwendung auf dem Gebiet der Medizintechnik und dort insbesondere zur Verwendung bei Infusionen, wobei unter einer Infusion allgemein die Zufuhr einer Flüssigkeit in den venösen oder arteriellen Teil des Kreislaufs eines Lebewesens verstanden wird.

Der Aufgabenbereich der Infusionstherapie ist groß und liegt im Wesentlichen auf den Gebieten einer Zufuhr von Kalorienträgern bei künstlicher Ernährung, einer Zufuhr von Medikamenten, der Regulierung des Elektrolyt-Haushalts und des Säure-Basen-Gleichgewichtes, der Flüssigkeitszufuhr zur forcierten Ausscheidung von Giftstoffen durch die Niere, beispielsweise bei Schlafmittelvergiftungen und dergleichen. Innerhalb eines Krankenhauses werden Infusionen hauptsächlich in der Intensivmedizin, in der Aufnahme und im Notfalldienst, bei Operationen, bei Entbindungen, bei der Säuglings- und Kinderpflege, bei der Funktionsdiagnostik sowie der Normalpflege verwendet. Eine Infusion kann manuell oder unter dem Einsatz von Infusionsapparaten durchgeführt werden.

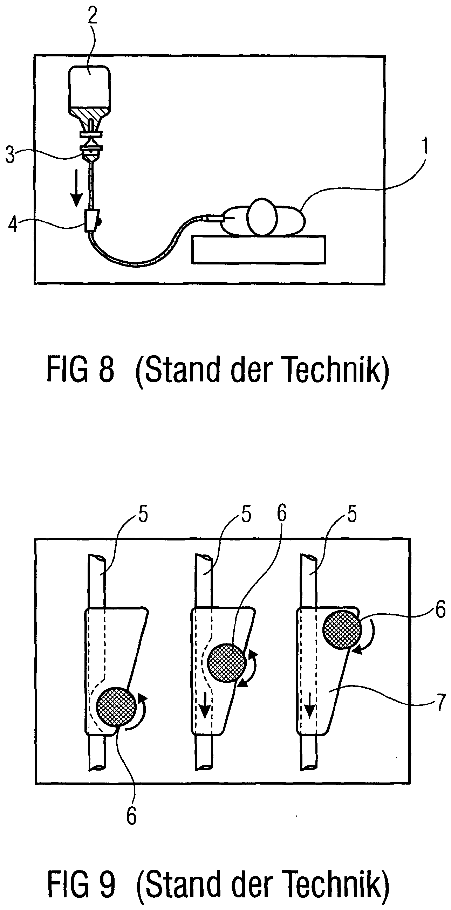

Für die Wahl der zweckmäßigen Infusionstechnik sind die geforderte Infusionsrate, die Infusionsdauer, die Dosiergenauigkeit und die erforderliche Applikationstechnik entscheidend. Am häufigsten eingesetzt wird die herkömmliche, manuelle Infusionstechnik, bei der es sich um eine Schwerkraftinfusion mit geringen Anforderungen an die Dosiergenauigkeit und die Dosierrate handelt. Ein Aufbau für eine solche Diffusion ist schematisch in Fig. 8 dargestellt. Die Flüssigkeitszufuhr wird lediglich durch das hydrostatische Druckgefälle zwischen einem Patienten 1 und einer in eine Flasche 2 gefüllten Infusionslösung bewirkt. Gegebenenfalls findet eine Unterstützung durch Kompression der Infusionslösung statt, was als manuelle Druckinfusion bezeichnet werden kann. Eine Tropfkammer 3 und eine Rollenschlauchklemme 4 sind in dem Fluidweg zwischen der Infusionsflasche 2 und dem Patienten 1 vorgesehen.

Bei einer solchen Anordnung ist die Infusion schwierig zu dosieren, da die Infusionsrate, d.h. die Geschwindigkeit der Flüssigkeitszufuhr, nur manuell durch Schließen oder Öffnen der Rollenschlauchklemme 4 reguliert werden kann, wie schematisch in Fig. 9 dargestellt ist, die drei Zustände einer Rollenschlauchklemme zeigt, wobei in der linken Darstellung ein Fluidschlauch 5 durch eine Rolle 6 der Rollenschlauchklemme vollständig abgeklemmt ist, in der mittleren Darstellung der Fluidschlauch 5 durch die Rolle teilweise abgeklemmt ist und in der rechten Darstellung der Fluidschlauch 5 durch die Rolle nicht abgeklemmt ist. Eine entsprechende Führung 7 ist vorgesehen, die eine Bewegung der Rolle 6 auf die in Fig. 9 gezeigte Weise ermöglicht, um unterschiedliche Abklemmzustände des Fluidschlauchs 5 zu realisieren.

Die Infusionsgeschwindigkeit ist von einer Vielzahl von Faktoren abhängig, die durch eine Rollenschlauchklemme nur schwer oder überhaupt nicht ausgeschaltet werden. Solche Faktoren sind u.a. die Form- und Fertigungsqualität, des Tropfenrohres in der Tropfenkammer 3 des Infusionsbestecks, die Tropfenbildungsgeschwindigkeit, die Konstanz des Förderdruckes, die physikalischen Eigenschaften der Infusionslösung und die Umgebungsbedingungen. Aufgrund dieser Faktoren können mit manuellen Infusionssystemen nur geringe Fördergenauigkeiten von ±20% erreicht werden, wobei Abweichungen von ±50% durchaus nicht unüblich sind.

Wegen dieser geringen Fördergenauigkeit findet die Schwerkraftinfusion dann Anwendung, wenn die Infusionstherapie dies zulässt. Darüber hinaus müssen natürlich die physikalischen Voraussetzungen, wie z.B. Druck und Förderrate, für eine Schwerkraftinfusion gegeben sein. Bei einer Schwerkraftinfusion erfolgt die Messung der Förderrate und eines Katheterverschlusses nur manuell durch Zählung der Tropfen in der Tropfenkammer. Bei sehr kleinen Dosierraten sind sehr lange Beobachtungszeiten nötig, um einen Verschluss zu erkennen. Außerdem ist die Herstellung einer Tropfenkammer ein Kostenfaktor.

Durch den Einsatz von Infusionsapparaten, die beispielsweise Spritzenpumpen verwenden, kann die Infusionstherapie bezüglich einer Erhöhung der Infusionsrate, einer Steigerung der Dosiergenauigkeit und einer Gewährleistung einer konstanten Förderung bei Langzeitinfusionstherapien verbessert werden. Durch diese Vorteile von Infusionsapparaten kann das Behandlungsspektrum von Infusionstherapien erweitert werden. Bei Infusionen mit Spritzenpumpen erfolgt die Messung der Fördermenge nur indirekt über den Vorschub des Spritzenmotors. Eine direkte Messung der Dosierrate erfolgt nicht, was ein Sicherheitsrisiko darstellt. Bei Infusionen mit Spritzenpumpen erfolgt die Detektion eines Katheterverschlusses in der Regel über eine Erfassung des Anstiegs des Motorstroms. Ein Verschluss des Katheters wird vor allem bei kleinen Fördermengen erst nach langen Verzögerungszeiten bis zu einer Stunde erkannt.

Eine Vorrichtung, um Fluid zu einem Patienten zu liefern, ist aus der US 2004/0127844 A1 bekannt. Die Vorrichtung umfasst einen Dispenser, eine Fluidleitung mit einem Ausgangsanschluss, der zum Koppeln beispielsweise mit einer Nadel geeignet ist, und einen Flusszustandssensor in der Flussleitung zwischen dem Dispenser und dem Ausgangsanschluss. Der Prozessor ist programmiert, um einen Fluidfluss zu dem Auslassanschluss zu bewirken. Der Flusszustandssensor überwacht Flussbedingungen in dem Fluidweg, die während des Betriebs auftreten können, um sicherzustellen, dass Fluid bestimmungsgemäß zugeführt wird. Der Flusszustandssensor weist eine Membran auf, die eine Fluidkammer teilweise begrenzt, so dass durch eine Auslenkung der Membran ein Druck in der Fluidkammer qualitativ bewertet werden kann. Eine quantitative Messung eines Drucks bzw. eine Bestimmung einer Strömungsrate finden nicht statt.

Aus der US 2007/0151346 A1 ist ein optisches Drucküberwachungssystem bekannt, das eine Leitung von einem Infusionsbesteck und einen optischen Signalsensor aufweist, der angeordnet ist, um Änderungen des Durchmessers der Leitung zu erfassen und dadurch Druckänderungen in der Leitung zu bestimmen. Jeweils ein solcher Sensor kann vor und hinter einer Rotorpumpe angeordnet sein. Eine vergleichbare Anordnung ist auch in der US 5,720,721 beschrieben.

Die US 4,994,035, EP-A1-1769738, EP-A1-1818664 und EP-A2-0401524 beschreiben jeweils Sensoren zur Ermittlung von Drücken im Fluidweg von Mikrodosiervorrichtungen, wie z.B. Infusionsleitungen.

Die WO96/03168 A offenbart eine Infusionspumpe, die eine fluidische Reihenschaltung aus einem Einlasssensor, einem Einlassventil, einem Verdränger, einem Auslassventil und einem Auslasssensor aufweist.

Die DE4308313 A1 offenbart eine Vorrichtung zur Bestimmung der Durchflussrate eines Mediums nach dem Differenzdruckprinzip, wobei ein Drosselschlauch zwischen eine erste Messkammer und eine zweite Messkammer geschaltet ist.

Die EP1772162 A befasst sich mit einem Infusionssystem zum Verabreichen eines flüssigen Medikaments, bei dem ein Drucksensor zum Überwachen eines in einem Infusionsschlauch herrschenden Flüssigkeitsdrucks vorgesehen ist.

Die Aufgabe der vorliegenden Erfindung besteht darin, Vorrichtungen zum Erfassen zumindest eines Strömungsparameters zu schaffen, die eine zuverlässige Dosierung mit einer gewünschten Dosierrate ermöglichen.

Diese Aufgabe wird durch eine Vorrichtung nach Anspruch 1 gelöst.

Ausführungsbeispiele der Erfindung schaffen ferner ein Fluidikmodul und ein Detektionsmodul für eine entsprechende Vorrichtung.

Ausführungsbeispiele der Erfindung basieren auf der Erkenntnis, dass die Verwendung zweier Flussrestriktionen und zweier jeweils hinter den Flussrestriktionen angeordneter Drucksensoren ermöglicht, sowohl eine Strömungsrate eines durch eine entsprechende Fluidikstruktur strömenden Fluids zu bestimmen, als auch Verstopfungen der jeweiligen Flussrestriktionen oder eines dem zweiten Messbereich folgenden Fluidbereichs sicher zu erfassen.

Unter "Flussrestriktion" wird dabei hierin jeweils eine Flussrestriktion verstanden, die einen definierten Flusswiderstand aufweist, d.h. bekannte geometrische Abmessungen aufweist, die zur Bestimmung der Dosierrate herangezogen werden können. Bei Ausführungsbeispielen der Erfindung sind die Flussrestriktionen so ausgelegt, dass bei im Normalbetrieb auftretenden Flüssen (d.h. ohne Verstopfungen) ein Druckabfall stattfindet, der bewirkt, dass der Druck im zweiten Messbereich geringer ist als der Druck im ersten Messbereich.

Bei Ausführungsbeispielen der Erfindung weisen die Messbereiche jeweils Membranabschnitte auf, die durch den in den Messbereichen herrschenden Druck ausgelenkt werden, so dass die druckabhängige Position der Membrane, die sich jeweils hinter einer Flussrestriktion mit definiertem Flusswiderstand befinden, gemessen werden kann.

Bei Ausführungsbeispielen der Erfindung können die mit dem strömenden Fluid in Berührung kommenden Teile der Vorrichtung als Wegwerfteil bzw. Einwegteil konzipiert sein, das austauschbar mit den übrigen Komponenten koppelbar ist. Dieses Einwegteil kann als Kunststoffteil mit sehr geringen Kosten konzipiert sein. Durch den erfindungsgemäßen Lösungsansatz können Tropfkammern überflüssig werden, so dass diese weggelassen werden können, so dass sich eine Kosteneinsparung des Einwegteiles gegenüber herkömmlichen Einweg-Infusionsbestecken ergeben kann.

Ausführungsbeispiele der Erfindung ermöglichen einen verzögerungsfreien Nachweis eines Verschlusses, auch bei kleinen Infusionsraten, insbesondere wenn die erfindungsgemäße Vorrichtung unmittelbar vor einem Patientenzugang, beispielsweise in Form einer Kanüle, vorgesehen wird. Bei Ausführungsbeispielen der Erfindung kann eine Alarmfunktion vorgesehen sein, um bei Erfassen einer Verstopfung Alarm auszulösen. Bei Ausführungsbeispielen der Erfindung kann eine Messung der Infusionsrate stattfinden, was im Vergleich zu einer Tropfenzählmethode wesentlich genauer ist, wobei eine Anzeige vorgesehen sein kann, die die ermittelte Strömungsrate anzeigt. Durch eine entsprechende Anzeige kann ein Pfleger ferner in die Lage versetzt werden, die Infusionsrate durch eine Rollenschlauchklemme exakt einzustellen, wodurch die Dosiergenauigkeit deutlich ansteigen kann.

Im Vergleich zur Spritzeninfusionstechniken können bei dem erfindungsgemäßen Lösungsansatz die Kosten des Einwegteiles deutlich reduziert sein. Bei Ausführungsbeispielen der Erfindung kann die Auswertungseinrichtung mit einer Pumpvorrichtung, beispielsweise einer Spritzenpumpe, kommunizieren, um diese zu steuern, um eine gewünschte Strömungsrate zu erhalten. Ferner kann die Auswerteeinrichtung ausgelegt sein, um auftretende Fehlzustände zu erfassen und/oder anzuzeigen. So kann ein Katheterverschluss verzögerungsfrei auch bei kleinen Infusionsraten nachgewiesen werden. Die Infusionsrate kann direkt gemessen werden, so dass durch die Pumpvorrichtung, wie z.B. die Spritzenpumpe, die Dosierrate entsprechend nachgeregelt werden kann, um eine gewünschte Dosierrate zu erreichen. Ferner kann bei Ausführungsbeispielen die Auswerteeinrichtung auch erkennen, wenn die Pumpeinrichtung ganz ausfällt.

Ausführungsbeispiele der erfindungsgemäßen Vorrichtungen sind selbstverständlich auch außerhalb der Medizintechnik auf anderen Gebieten einsetzbar, wo eine Fluidströmung mit einer definierten Strömungsrate erforderlich ist.

Ausführungsbeispiele der vorliegenden Erfindung werden nachfolgend Bezug nehmend auf die beiliegenden Zeichnungen näher erläutert. Es zeigen:

Fig. 1 eine schematische Darstellung eines Ausführungsbeispiels einer erfindungsgemäßen Vorrichtung;

Fig. 2 eine schematische Darstellung eines alternativen Ausführungsbeispiels;

Fig. 3a, 3b schematische Darstellungen eines Ausführungsbeispiels eines erfindungsgemäßen Fluidikmoduls;

Fig. 4a bis 4c schematische Darstellungen von Drucksensoren;

Fig. 5 eine schematische Darstellung zur Veranschaulichung der Funktionsweise von Ausführungsbeispielen der Erfindung;

Fig. 6 eine graphische Darstellung der Temperaturabhängigkeit der Viskosität unterschiedlicher Medien;

Fig. 7 eine schematische Darstellung eines weiteren Ausführungsbeispiels der Erfindung;

Fig. 8 eine schematische Darstellung einer Schwerkraftinfusion nach dem Stand der Technik; und

Fig. 9 eine schematische Darstellung einer Rollenschlauchklemme nach dem Stand der Technik.

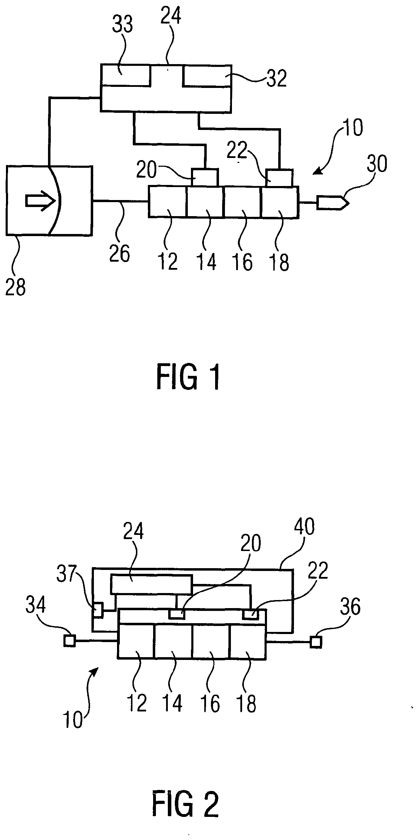

Fig. 1 zeigt schematisch ein Ausführungsbeispiel einer Vorrichtung zum Erfassen zumindest eines Strömungsparameters. Die Vorrichtung umfasst eine Reihenschaltung 10 einer Mehrzahl von Fluidbereichen, die eine erste Fluidrestriktion 12, einen der ersten Fluidrestriktion 12 strömungsmäßig nachgeschalteten ersten Messbereich 14, eine dem ersten Messbereich 14 strömungsmäßig nachgeschaltete zweite Fluidrestriktion 16 und einen der zweiten Fluidrestriktion 16 strömungsmäßig nachgeschalteten zweiten Messbereich 18 aufweist. Dem ersten Messbereich 14 ist ein erster Sensor 20 zugeordnet, und dem zweiten Messbereich 18 ist ein zweiter Sensor 22 zugeordnet. Die Sensoren 20 und 22 sind ausgelegt, um ein Maß für den in den zugeordneten Messbereichen 14 und 18 herrschenden Druck zu erfassen, beispielsweise durch Erfassen der Auslenkung einer jeweiligen Membran, die an den Messbereich 14 und den Messbereich 18 angrenzt. Die Sensoren 20 und 22 sind kommunikativ (drahtlos oder drahtgebunden) mit einer Auswerteeinrichtung 24 verbunden, die, wie für einen Fachmann klar ist, durch eine Mikroprozessoreinrichtung, eine ASIC (anwendungsspezifische integrierte Schaltung) oder dergleichen implementiert sein kann.

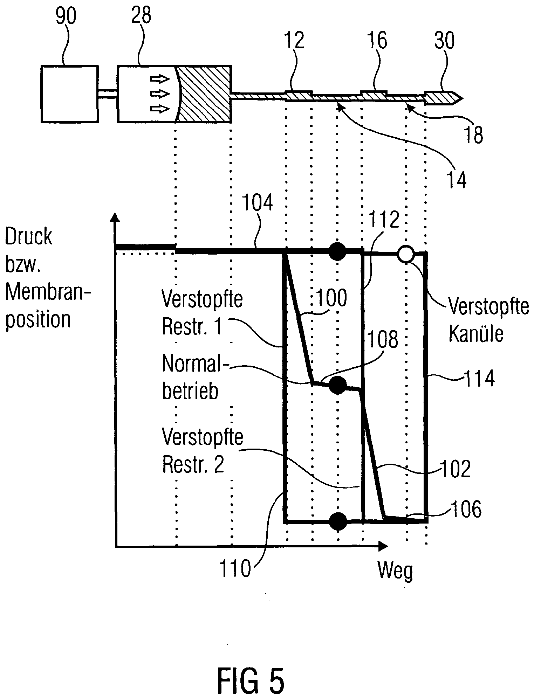

Die Reihenschaltung 10 ist fluidisch in eine Fluidleitung 26 geschaltet, die eine Pumpeinrichtung 28 mit einer Kanüle 30 fluidisch verbindet. Die Kanüle 30 kann beispielsweise eine Infusionsnadel zum Legen einer Infusion für einen Patienten sein. Die Auswertungseinrichtung empfängt von den Sensoren 20 und 22 die Maße für den Druck in den Messbereichen 14 und 18 und kann ausgelegt sein, um basierend darauf eine Strömungsrate durch die Reihenschaltung 10 und somit durch die Fluidleitung 26 zu bestimmen. Die Auswerteeinrichtung 24 kann ferner ausgelegt sein, um basierend auf den Ausgangssignalen der Sensoren 20 und 22 Verstopfungsbedingungen festzustellen, wie nachfolgend Bezug nehmend auf Fig. 5 näher erläutert wird. Die Auswerteeinrichtung 24 kann eine Anzeigevorrichtung 32 zum Anzeigen der Strömungsrate und/oder einer Verstopfung aufweisen. Die Auswerteeinrichtung 24 kann ferner eine Alarmeinrichtung (nicht gezeigt) aufweisen, um eine Verstopfung anzuzeigen.

Wie in Fig. 1 gezeigt ist, kann die Auswerteeinrichtung 24 ferner mit der Pumpeinrichtung 28 kommunikativ verbunden sein (entweder drahtlos oder drahtgebunden), um unter Verwendung der ermittelten Strömungsrate durch die Fluidleitung 26 die Pumpeinrichtung 28 zu steuern, um eine gewünschte Strömungsrate durch die Fluidleitung 26 und somit die Kanüle 30 zu erhalten. Alternativ könnte eine Rollenschlauchklemme (in Fig. 1 nicht gezeigt) vorgesehen sein, über die, unter Verwendung der bestimmten angezeigten Strömungsrate, ein Benutzer eine gewünschte Strömungsrate einstellen kann.

Fig. 2 zeigt eine schematische Ansicht eines Ausführungsbeispiels einer Vorrichtung zum Bestimmen zumindest eines Strömungsparameters, bei dem die mit dem strömenden Fluid in Berührung kommenden Teile als Einwegartikel aufgebaut sind. Diese Teile sind die fluidische Reihenschaltung 10, an deren Einlassende und Auslassende fluidische Anschlüsse 34 und 36 vorgesehen sind, die dazu dienen, die Reihenschaltung 10 beispielsweise in eine Fluidleitung zwischen einer Pumpeinrichtung und einem Patienten zu schalten.

Das beschriebene Einwegmodul, das die mit dem strömenden Fluid in Berührung kommenden Teile umfasst, kann austauschbar mit einem Detektormodul 40 gekoppelt werden, das die Sensoren 20 und 22, die mit der Auswertungseinrichtung 24 gekoppelt sind, aufweist. Das Einwegmodul und das Detektormodul 40 sind auf geeignete Weise verbindbar, derart, dass die Sensoren 20 und 22 bezüglich der Messbereiche 14 und 18 richtig positioniert werden, um ein Maß für den in den Messbereichen herrschenden Druck erfassen zu können. Entsprechende Ausrichtungsmittel, um eine solche Anordnung zu erreichen, können sowohl an dem Detektormodul als auch an dem Einwegmodul, das die Fluidbereiche aufweist, vorgesehen sein. Ferner können geeignete Schnellverbindungsmittel, wie Schrauben oder Klemmen vorgesehen sein, um die Module austauschbar aneinander zu befestigen.

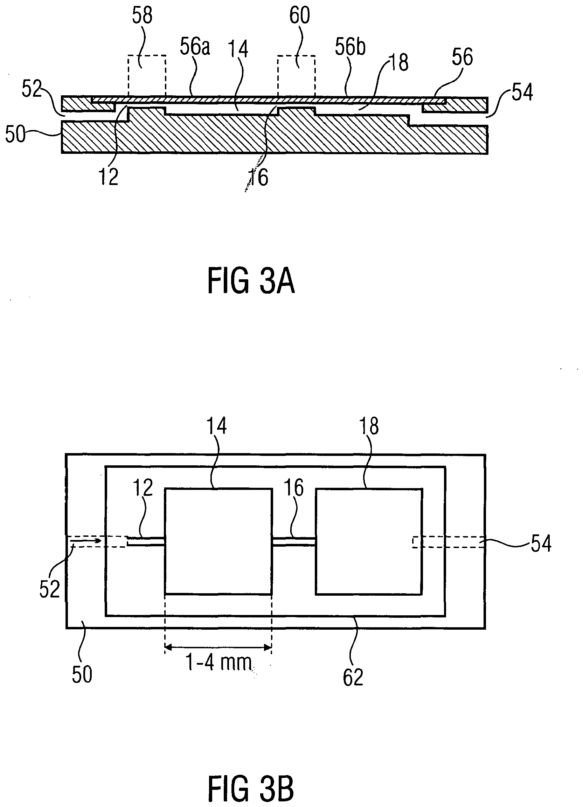

Bei Ausführungsbeispielen der Erfindung erfolgt die Erfassung des Maßes für den Druck in den Messbereichen durch die Messung der druckabhängigen Position bei zwei ausgelenkten Membranen, die sich jeweils hinter einer Flussrestriktion mit definiertem Flusswiderstand befinden. Ein Ausführungsbeispiel für ein entsprechendes Modul, das eine entsprechende Reihenschaltung von Fluidbereichen aufweist, ist in den Fig. 3a und 3b gezeigt, wobei Fig. 3a eine Querschnittsdarstellung und Fig. 3b eine Draufsicht darstellt.

Das in Fig. 3a gezeigte Fluidikmodul umfasst einen einstückigen Modulkörper 50, in dem eine Zuleitung 52 und eine Ableitung 54 gebildet sind. Ferner ist die obere Oberfläche des Modulkörpers 50 strukturiert, um zusammen mit einer Membran 56 die erste Flussrestriktion 12, den ersten Messbereich 14, die zweite Flussrestriktion 16 und den zweiten Messbereich 18 zu definieren. Die Membran 56 kann, wie in Fig. 3a gezeigt ist, in eine Ausnehmung in der oberen Oberfläche des Modulkörpers 50 eingebracht sein. Oberhalb der Messbereiche 14 und 18 angeordnete Membranabschnitte 56a und 56b der Membran 56 sind abhängig von einem in den Messbereichen 14 und 18 herrschenden Druck auslenkbar, so dass durch eine Erfassung der Position des Membranabschnittes 56a ein Maß für den Druck in der Messkammer 14 erfasst werden kann und durch ein Erfassen der Position des Membranabschnittes 56b eine Maß für den Druck in der Messkammer 18 erfasst werden kann. Um ein Auslenken der Membran im Bereich der Flussrestriktionen 12 und 16 zu verhindern und somit einen konstanten Strömungswiderstand der Flussrestriktionen sicherzustellen, kann das Detektormodul, mit dem das in Fig. 3a gezeigte Fluidikmodul austauschbar verbunden wird, Gegenhalteelemente aufweisen, die in Fig. 3a durch gestrichelte Linien angedeutet und mit den Bezugszeichen 58 und 60 bezeichnet sind. Alternativ können die Membranen in dem Bereich der Flussrestriktionen 12 und 16 auslenkbar sein, so dass der Strömungsquerschnitt der Flussrestriktionen druckabhängig ist, was den Vorteil hat, dass bei gleichem Druckabfall der Strömungsbereich zu höheren Flüssen hin erweitert werden kann.

Bei der Draufsicht in Fig. 3b ist die Membran 56 weggelassen, um die darunter liegenden Strukturen nicht zu verdecken, wobei die Ausnehmung in dem Modulkörper 50, in den die Membran eingebracht ist, mit dem Bezugszeichen 62 bezeichnet ist.

Die Strömungswiderstände der Flussrestriktionen 12 und 16 können auch unterschiedlich dimensioniert sein, d.h. mit unterschiedlichen Strömungsquerschnitten und Restriktionslängen.

Die Flussrestriktionen sind derart ausgelegt, dass bei den im Normalbetrieb auftretenden Flüssen ein Druckabfall stattfindet, so dass aufgrund des Druckabfalls über der Flussrestriktion 16 der zweite Membranabschnitt 56b weniger stark ausgelenkt wird, wie der erste Membranabschnitt 56a. Die Messbereiche 14 und 18 weisen im Vergleich zu den Flussrestriktionen einen solchen Flussquerschnitt auf, dass ein Druckabfall über denselben vernachlässigbar ist. Beispielsweise können die Flusswiderstände so ausgelegt sein, dass ein Druckabfall über jede der Flussrestriktionen im Normalbetrieb zumindest 10-mal bis 200-mal größer ist als ein Druckabfall über jede der Messkammern.

Bei Ausführungsbeispielen der Erfindung kann der Modulkörper 50 aus Kunststoff gebildet sein. Die elastische Membran kann aus einem beliebig geeigneten Material gebildet sein, beispielsweise Silikon oder Gummi, und kann beispielsweise durch Laserschweißen, Thermokompressionsschweißen, Ultraschallschweißen, Lösemittelkleben oder anderweitiges Kleben an den Modulkörper gefügt sein. Der Zulauf 52 und der Ablauf 54 können ausgebildet sein, um auf einfache Weise mit Anschlussleitungen verbunden zu werden, beispielsweise Infusionsleitungen. Entsprechende Anschlusseinrichtungen, z.B. LUER-Verbinder, können vorgesehen sein.

Das Fluidikmodul kann somit als kostengünstiges Wegwerfteil implementiert sein und ermöglicht, wie im Folgenden erläutert wird, sowohl die Ermittlung einer Strömungsrate als auch das Erkennen von Verstopfungen, indem die Auslenkungen der beiden Membranabschnitte 56a und 56b erfasst werden. Lediglich um einen Hinweis auf Größenverhältnisse bei Ausführungsbeispielen der Erfindung zu geben, kann die Länge der Messkammer 14 1 bis 4 mm betragen, wie in Fig. 2b angedeutet ist.

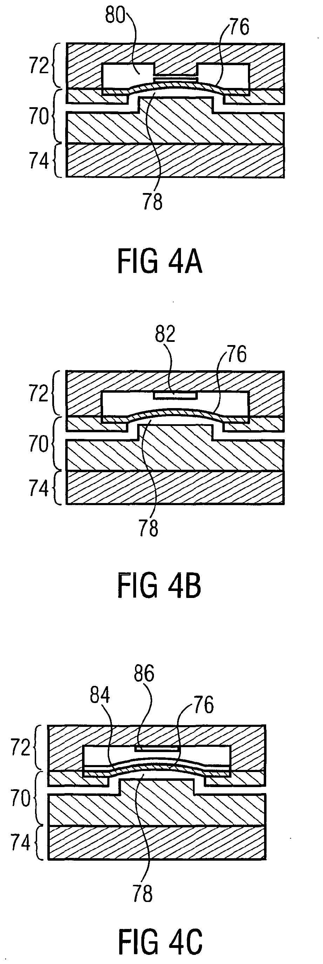

Bei Ausführungsbeispielen der Erfindung kann die Membranposition durch ein Detektionsmodul gemessen werden, dass wiederverwertbar sein kann. Die Messung der Position kann dabei auf unterschiedliche Arten erfolgen, wobei drei beispielhafte Möglichkeiten in den Fig. 4a bis 4c gezeigt sind. Die Fig. 4a bis 4c zeigen jeweils schematisch Abschnitte eines Fluidikmoduls, die mit dem Bezugszeichen 70 versehen sind. Die Abschnitte 70 können Teil eines Wegwerfmoduls sein, während die mit den Bezugszeichen 72 und 74 bezeichneten Abschnitte Teile eines wiederverwertbaren Detektormoduls sein können. Die Fig. 4a bis 4c sind lediglich veranschaulichend für Möglichkeiten zur Erfassung der Position einer jeweiligen Membran 76, die über einer Messkammer 78 angeordnet ist, so dass die Auslenkung der Membran 76 ein Maß für einen in der Messkammer 78 herrschenden Druck ist.

Gemäß Fig. 4a erfolgt die Erfassung über mechanische Schalter, die ausgebildet sind, um ein quantitatives Maß für die Auslenkung der Membran zu erhalten.

Gemäß Fig. 4b ist ein optischer Sensor 82, beispielsweise in der Form einer Reflexionslichtschranke, vorgesehen, um die Auslenkung der Membran 76 zu erfassen.

Gemäß Fig. 4c erfolgt eine kapazitive Messung der Auslenkung der Membran, wobei zu diesem Zweck eine Elektrodenschicht 84 auf der elastischen Membran 76 vorgesehen ist und wobei auf dem Detektormodulabschnitt 72 eine Gegenelektrode 86 vorgesehen ist, so dass die beiden Elektroden eine kapazitive Messung ermöglichen.

Bei Ausführungsbeispielen der Erfindung ermöglichen somit die Sensoren die Erfassung einer Auslenkung der Membranabschnitte 56a und 56b als Maß für die in den Messkammern 14 und 18 herrschenden Drücke.

Der Zusammenhang zwischen dem herrschenden Druck unter den Messmembranen und den Auslenkungen der Membran ist bei kleinen Drücken proportional zum Druck, bei größeren Auslenkungen können sich Abweichungen von der Linearität ergeben. Diese Funktion zwischen Druck und Auslenkung bleibt jedoch immer stetig monoton steigend, und kann somit einfach Chargen- oder Einzel-kalibriert und im Detektionsmodul elektronisch verarbeitet werden.

Bei einer Chargen- oder Einzel-Kalibrierung der Wegwerfmodule können die Kalibrierparameter auf dem Wegwerfteil dokumentiert werden, z.B. mit einem Strichcode, und dann bei der Montage mit dem Detektionsmodul von diesem, beispielsweise optisch, ausgelesen werden.

Bezug nehmend auf Fig. 5 wird nun erläutert, wie ausgehend von dem erfassten Druck bzw. der erfassten Membranposition auf mögliche Verstopfungen rückgeschlossen werden kann. Zu diesem Zweck sind im oberen Bereich von Fig. 5 die Pumpvorrichtung 28 und die fluidische Reihenschaltung aus erster Flussrestriktion 12, erster Messkammer 14, zweiter Flussrestriktion 16 und zweiter Messkammer 18 schematisch dargestellt. Ferner ist zu erkennen, dass der zweite Messbereich 18 unmittelbar angrenzend an eine Kanüle 30 vorgesehen sein kann. Schließlich ist schematisch ein Fluidreservoir 90, das zum Auffüllen der Pumpeinrichtung 28 dienen kann, schematisch in Fig. 5 gezeigt.

Im unteren Bereich von Fig. 5 sind der Druck bzw. die Membranpositionen für unterschiedliche Betriebszustände dargestellt.

Im Normalbetrieb, d.h. es liegt keine Verstopfung vor, findet über die erste Flussrestriktion 12 ein Druckabfall 100 und über die zweite Flussrestriktion 16 ein Druckabfall 102 statt. Somit herrscht im Messbereich 14 ein Druck, der zwischen einem vor der ersten Flussrestriktion 12 herrschenden Druck 104 und einem in dem zweiten Messbereich 18 herrschenden Druck 106 liegt. Sind die Flussrestriktionen 12 und 16 gleich, so liegt der in dem ersten Messbereich 14 herrschende Druck 108 mittig zwischen den Drücken 104 und 106.

Wenn die erste Flussrestriktion 12 verstopft ist, so ist der Druck in beiden Messbereichen 14 und 18 identisch auf einem geringen Pegel, der im Wesentlichen dem Pegel 106 entspricht, siehe Kurve 110 in Fig. 5. Ist die Flussrestriktion 14 verstopft, so ist der Druck in dem Messbereich 18 im Wesentlichen auf dem niedrigen Pegel 106, während der Druck in dem Messbereich 14 im Wesentlichen auf dem hohen Pegel 104 ist, siehe Kurve 112 in Fig. 5. Ist die Kanüle 30 verstopft, so sind beide Messkammern 14 und 18 im Wesentlichen auf dem hohen Pegel 104, siehe Kurve 114 in Fig. 5. Ein Ausfall der Pumpeinrichtung hätte, wie eine Verstopfung der Restriktion 1, zur Folge, dass der Druck in den beiden Messkammern 14 und 18 im Wesentlichen auf dem niedrigen Pegel 106 ist.

Die erfindungsgemäße Anordnung von Flussrestriktionen und Messbereichen ermöglicht somit zuverlässig die Erkennung aller Verstopfungszustände. Insbesondere lässt sich auch eine Verstopfung des Patientenzugangs verzögerungsfrei erkennen, wenn die Vorrichtung unmittelbar vor einem Patientenzugang montiert wird.

Die Auswertungseinrichtung kann beispielsweise ausgebildet sein, um die erfassten Druckmaße miteinander und/oder mit Sollwerten zu vergleichen, um basierend auf dem Ergebnis solcher Vergleiche auf den vorliegenden Strömungszustand rückzuschließen. Sind beide Maße auf einem hohen Pegel, bestimmt die Auswertungseinrichtung eine Verstopfung der Kanüle. Sind beide Maße auf einem tiefen Pegel, so bestimmt die Auswertungseinrichtung eine Verstopfung der ersten Flussrestriktion. Ist das erste Maß auf einem hohen Pegel und das zweite Maß auf einem tiefen Pegel, so bestimmt die Auswertungseinrichtung eine Verstopfung der zweiten Flussrestriktion. Ist das erste Maß auf einem mittleren Pegel und das zweite Maß auf einem tiefen Pegel, so bestimmt die Auswertungseinrichtung keine Verstopfung, da ein Normalbetrieb erkannt wird.

Weiterhin ermöglicht die erfindungsgemäße Anordnung die Bestimmung eines quantitativen Maßes für die Strömung, wenn die Differenz der Membranpositionen, die durch die Sensoren 20 und 22 erfasst wird, bestimmt wird, da der Strömungswiderstand der Flussrestriktion, bzw. die geometrischen Abmessungen derselben, zwischen den beiden Sensoren bekannt ist. Die Strömungsrate bzw. Dosierrate Q bestimmt sich zu:

$$Q = \frac{1}{C_R} \cdot \frac{A^2}{\eta L} \cdot \Delta p$$

$C_R$ stellt den Formfaktors des Querschnittes der zweiten Flussrestriktion dar, A stellt den Querschnitt der zweiten Flussrestriktion dar, η stellt die Viskosität des strömenden Fluids dar, L stellt die Länge der zweiten Flussrestriktion dar, und Δp stellt den Druckabfall über die zweite Flussrestriktion dar. Der Druckabfall Δp ergibt sich aus den von den Sensoren 20 und 22 erfassten Maßen für die Drücke in den Messbereichen 14 und 18. Der Formfaktor $C_R$, der Querschnitt A und die Länge L sind somit durch die Geometrie der zweiten Flussrestriktion festgelegt und bekannt und der Druckabfall Δp kann beispielsweise aus durch Reflexionslichtschranken gemessene Membranpositionen bestimmt werden. Bei einer reproduzierbaren Herstellung der Geometrie der zweiten Flussrestriktion kann somit die Strömungsrate zuverlässig und exakt bestimmt werden.

Der Messbereich des Strömungssensors kann in weiten Bereichen einfach angepasst werden, indem die Querschnittsfläche A der zweiten Flussrestriktion variiert wird. Es kann bevorzugt sein, dass der Flusswiderstand der ersten Flussrestriktion dem Flusswiderstand der zweiten Flussrestriktion entspricht, so dass der zwischen den Flussrestriktionen herrschende Druck mittig zwischen den vor und hinter den Restriktionen herrschenden Drücken liegt. Bei einem anderen Verhältnis der Flusswiderstände der Flussrestriktionen zueinander verschiebt sich dieser Druck entsprechend zu dem einen oder anderen Druck hin.

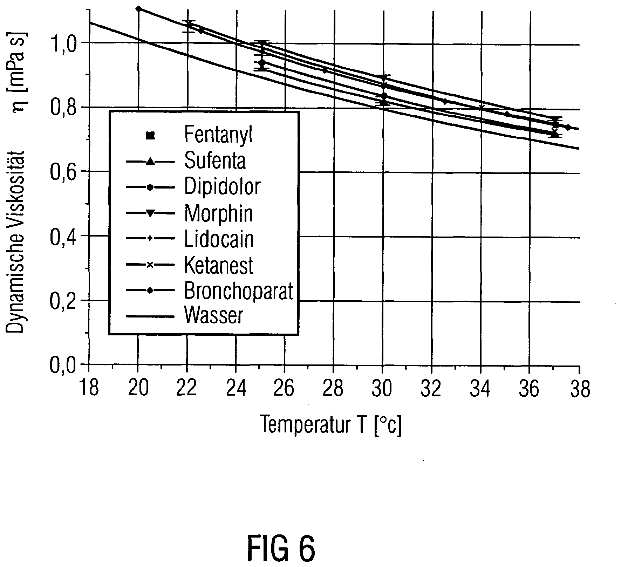

Bei Ausführungsbeispielen der vorliegenden Erfindung kann die Auswertungseinrichtung ausgebildet sein, um die Strömungsrate eines durch die Reihenschaltung strömenden Fluids auf der Grundlage der erfassten Druckmaße sowie auf der Grundlage der Geometrie der zweiten Flussrestriktion zu ermitteln. Bei Ausführungsbeispielen der Erfindung kann die Auswerteeinrichtung ausgelegt sein, um dabei unterschiedliche Viskositäten unterschiedlicher Medien zu berücksichtigen. Zu diesem Zweck kann die Viskosität unterschiedlicher Medien als Datensatz in einem Speicher des Detektionsmoduls bzw. der Auswerteeinrichtung hinterlegt sein, wie sie beispielsweise für eine Reihe von Medikamenten gemessen wurde. Die Viskosität von Medikamenten ist nicht nur leicht unterschiedlich, sondern auch temperaturabhängig. Bei Ausführungsbeispielen der Erfindung kann somit das Detektionsmodul zusätzlich einen Temperatursensor enthalten, der die Temperaturabhängigkeit der Viskosität kompensiert, damit die korrekte Dosierrate berechnet werden kann. Zu diesem Zweck können in dem Speicher die Viskositäten der verschiedenen Medien für unterschiedliche Temperaturen abgelegt sein, wobei die Auswerteeinrichtung abhängig von der von dem Temperatursensor erfassten Temperatur auf die am besten passende der abgelegten Viskositäten zur Ermittlung der Strömungsrate zurückgreift. Fig. 6 zeigt ein Diagramm, das die dynamische Viskosität unterschiedlicher Medikamente sowie Wasser für unterschiedliche Temperaturen zeigt. Die entsprechenden Werte können beispielsweise in tabellarischer Form für einen Zugriff durch die Auswertungseinrichtung in einem Speicher abgelegt sein.

Diesbezüglich zeigt Fig. 2 schematisch einen Temperatursensor 37, der in dem Detektormodul 40 angeordnet ist. Der Temperatursensor 37 kann möglichst nahe an fluidführenden Bereichen des Fluidikmoduls angeordnet sein, um möglichst exakt die Temperatur des strömenden Fluids zu erfassen. Ferner zeigt Fig. 1 schematisch einen Speicher 33, in dem Daten, die Viskositäten unterschiedlicher Medien widerspiegeln. Ferner können in dem Speicher Daten, die die Temperatur verschiedener Medien für unterschiedliche Temperaturen widerspiegeln, abgelegt sein.

Ausführungsbeispiele der Erfindung umfassen ein Einweg-Fluidikmodul und ein Mehrweg-Detektionsmodul, das wiederverwendbar ist. Folgende Punkte beeinflussen die Messgenauigkeit und bedürfen somit der Beachtung. So sollte bei der Produktion des Fluidikmoduls darauf geachtet werden, dass die Strömungswiderstände der Flussrestriktionen und die Membranelastizitäten eine geringe Streuung von Element zu Element haben, wobei eine Kalibrierung des Fluidikmoduls bei der Herstellung erfolgen kann. Um eine Einzelkalibrierung zu umgehen, kann z.B. die Integration von hochpräzisen und reproduzierbar herstellbaren Glaskapillaren oder in Silizium geätzten und gedeckelten Kanälen als Flussrestriktionen in das Fluidikmodul erfolgen. Beim Zusammenfügen des Fluidikmoduls in das Detektionsmodul ist darauf zu achten, dass die Sensoren, beispielsweise die Reflexionslichtschranken, einen definierten Abstand zu den elastischen Membranen haben. Das Detektionsmodul kann derart ausgelegt sein, dass es ein einfaches Einlegen des Fluidikmoduls, das als einfaches Kunststoffteil ausgebildet sein kann, ermöglicht, wobei Ausrichtungsmittel vorgesehen sein können, um sicherzustellen, dass der definierte Abstand erhalten wird.

Bei Ausführungsbeispielen der Erfindung können die elastischen Membranen so ausgelegt werden, dass sie bei gegebenen Druckabfällen maximal eine Auslenkung haben, die dem gesamten Messbereich einer Reflexionslichtschranke entspricht, beispielsweise 1 mm. Somit kann die Messauflösung für den Durchfluss maximal werden.

Um zu ermöglichen, dass der Strömungsquerschnitt der Flussrestriktionen sich nicht durch den angelegten Druck vergrößert, kann das Detektionsmodul einen mechanischen Anschlag über den Restriktionen aufweisen, der die Aufwölbung der elastischen Membran verhindert, wie oben Bezug nehmend auf Fig. 3a erläutert wurde. Alternativ könnte die Membran über der Restriktion aus einem nicht elastischen Material hergestellt werden, was jedoch Nachteile hinsichtlich eines höheren Montageaufwands, höherer Kosten und einer Leckgefahr mit sich bringt.

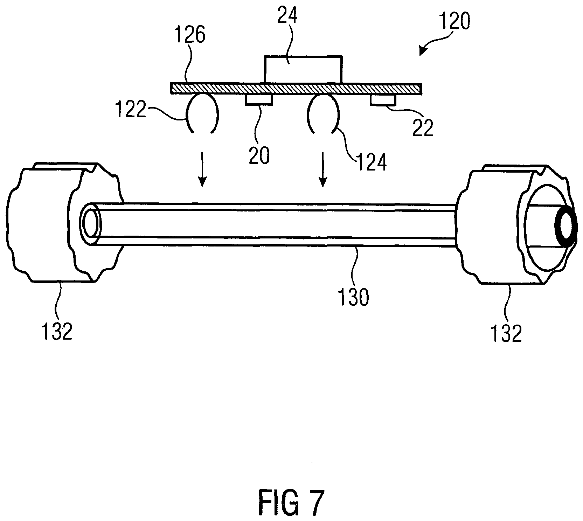

Ein mögliches Ausführungsbeispiel für ein Detektionsmodul ist in Fig. 7 gezeigt. Das Detektionsmodul 120 weist zwei Klammern 122 und 124 und zwei Sensoren 20 und 22 auf, die zusammen mit einer Auswerteeinrichtung 24 an einem Träger 126 angebracht sein können, der in Fig. 7 schematisch als Platte 126 dargestellt ist. Das Detektionsmodul 120 kann auf eine fluidische Leitung 130, wie z.B. einen Schlauch mit elastischen Wänden, aufgesteckt werden. Die fluidische Leitung 130 kann an beiden Enden derselben Anschlussmittel 132 aufweisen. Die Klammern 122 und 124 sind derart ausgebildet, dass durch das Aufstecken der Schlauch durch die Klammern definiert zusammengedrückt wird, so dass durch die Klammern 122 und 124 die erste und die zweite Flussrestriktion gebildet werden. Durch das Aufstecken werden ferner die Sensoren 20 und 22, die wiederum als Reflexionslichtschranken ausgebildet sein können, derart bezüglich der Wandung der fluidischen Leitung positioniert, dass Auslenkungen der Wandung erfasst werden können. Durch das Aufstecken des Detektionsmoduls 120 auf die Fluidleitung 130 ergibt sich somit wiederum eine fluidische Reihenschaltung aus erster Flussrestriktion, erstem Messbereich, zweiter Flussrestriktion und zweitem Messbereich.

Alternativ können Restriktionen und Sensoren jeweils als Einzelmodule ausgebildet sein, die fluidisch zusammengesteckt werden.

Ausführungsbeispiele der Erfindung beziehen sich auf die Verwendung erfindungsgemäßer Verfahren und Vorrichtungen bei einer Infusion, wie z.B. einer Schwerkraftinfusion oder einer Spritzeninfusion. Ausführungsbeispiele der vorliegenden Erfindung können somit durch ein entsprechendes Infusionsgerät implementiert sein.

Bei Ausführungsbeispielen der Erfindung können die Sensoren (20, 22) optische Sensoren, kapazitive Sensoren oder mechanische Schalter aufweisen.

Bei Ausführungsbeispielen des erfindungsgemäßen Fluidikmoduls ist ein Einlasskanal, der mit der ersten Flussrestriktion fluidisch verbunden ist, und ein Auslasskanal (54) der mit der zweiten Messkammer fluidisch verbunden ist, in dem Modulkörper (50) gebildet. Bei Ausführungsbeispielen können der Einlasskanal (52) und der Auslasskanal (54) in sich gegenüberliegenden Seitenflächen des Modulkörpers (50) enden.

Bei Ausführungsbeispielen des erfindungsgemäßen Verfahrens wird beim Bestimmen der Strömungsrate eine Temperatur und eine Temperaturabhängigkeit der Viskosität des Fluids berücksichtigt.

Bei Ausführungsbeispielen des erfindungsgemäßen Verfahrens wird bestimmt, dass eine Verstopfung der ersten Flussrestriktion vorliegt, wenn das erste Maß und das zweite Maß einen ersten geringen Druckpegel anzeigen, bei dem bestimmt wird, dass eine Verstopfung der zweiten Flussrestriktion vorliegt, wenn das erste Maß einen zweiten hohen Druckpegel anzeigt, der höher als der erste geringe Pegel ist, und das zweite Maß den geringen ersten Druckpegel anzeigt, bei dem bestimmt wird, dass eine Verstopfung des an den zweiten Messbereichs angrenzenden Fluidbereichs vorliegt, wenn das erste und das zweite Maß den zweiten hohen Druckpegel anzeigen, und bei dem bestimmt wird, dass keine Verstopfung vorliegt, wenn das erste Maß einen zwischen dem ersten und dem zweiten Druckpegel liegenden dritten Druckpegel anzeigt, und das zweite Maß einen Druckpegel anzeigt, der im Bereich des geringen ersten Druckpegels liegt.

Bei Ausführungsbeispielen umfasst das erfindungsgemäße Verfahren ferner ein Steuern einer Pumpeinrichtung unter Verwendung der erfassten ersten und zweiten Maße, um eine gewünschte Strömungsrate durch die fluidische Reihenschaltung zu bewirken.

**Patentansprüche**

1. Vorrichtung zum Bestimmen zumindest eines Strömungsparameters, mit:

    einer fluidischen Reihenschaltung (10), die in dieser Reihenfolge eine erste Flussrestriktion (12), einen ersten Messbereich (14), eine zweite Flussrestriktion (16) und einen zweiten Messbereich (18) aufweist,

    einem ersten Sensor (20) zum Erfassen eines quantitativen ersten Maßes für den Druck in dem ersten Messbereich (14);

    einem zweiten Sensor zum Erfassen eines zweiten quantitativen Maßes für den Druck in dem zweiten Messbereich (18); und

    einer Auswertungseinrichtung (24), die ausgelegt ist, um unter Verwendung der durch den ersten und zweiten Sensor (20, 22) erfassten Maße zu bestimmen, ob eine Verstopfung der ersten Flussrestriktion (12), der zweiten Flussrestriktion (16) oder eines sich an den zweiten Messbereich anschließenden Fluidbereichs vorliegt,

    **dadurch gekennzeichnet, dass** die Auswerteeinrichtung ausgelegt ist, um zu bestimmen,

    dass eine Verstopfung der ersten Flussrestriktion (12) vorliegt, wenn das erste Maß und das zweite Maß einen Druck anzeigen, der einen ersten geringen Pegel aufweist,

    dass eine Verstopfung der zweiten Flussrestriktion (16) vorliegt, wenn das erste Maß einen Druck mit einem zweiten hohen Pegel, der höher als der erste geringe Pegel ist, aufweist, und das zweite Maß einen Druck des geringen ersten Pegels anzeigt,

    dass eine Verstopfung des an den zweiten Messbereich (18) angrenzenden Fluidbereichs vorliegt, wenn das erste und das zweite Maß einen Druck des zweiten hohen Pegels anzeigen, und

    dass keine Verstopfung vorliegt, wenn das erste Maß einen zwischen dem ersten und dem zweiten Pegel liegenden dritten Druck anzeigt und das zweite Maß einen Druck auf einem Pegel anzeigt, der im Bereich des geringen ersten Pegels liegt.

2. Vorrichtung nach Anspruch 1, bei der die Auswertungseinrichtung (24) ferner ausgelegt ist, um unter Verwendung der durch den ersten und zweiten Sensor (20, 22) erfassten Maße eine Strömungsrate eines durch die Reihenschaltung strömenden Fluids zu bestimmen, wobei die Vorrichtung ferner eine Pumpeinrichtung (28) zum Bereitstellen einer Fluidströmung durch die fluidische Reihenschaltung (10) aufweist, wobei die Auswertungseinrichtung (24) ausgelegt ist, um die Pumpeinrichtung (28) basierend auf den erfassten Maßen zu steuern, um eine gewünschte Strömungsrate zu erhalten.

3. Vorrichtung nach Anspruch 1 oder 2, bei der der sich an den zweiten Messbereich anschließende Fluidbereich eine Kanüle (30) ist.

**4.** Vorrichtung nach einem der Ansprüche 1 bis 3, bei der die Messbereiche (14, 18) elastische Wandbereiche (56a, 56b) aufweisen, deren Position von den in den Messbereichen (14, 18) herrschenden Drücken abhängen, wobei die Sensoren (20, 22) ausgelegt sind, um die Position der elastischen Wandbereiche (56a, 56b) zu messen.

**5.** Vorrichtung nach einem der Ansprüche 1 bis 4, bei der die fluidische Reihenschaltung (10) Strukturen in einem Fluidikmodulkörper (50), die zumindest in den Messbereichen (14, 18) von einer flexiblen Membran (56) abgedeckt sind, aufweisen.

**6.** Vorrichtung nach Anspruch 5, bei dem die flexible Membran (56) sich über die Flussrestriktionen (12, 16) erstreckt, wobei Gegenhalteelemente (58, 60) vorgesehen sind, um ein Auslenken der flexiblen Membran im Bereich der Flussrestriktionen zu verhindern.

**7.** Vorrichtung nach einem der Ansprüche 1 bis 6, die ferner einen Temperatursensor (37) aufweist, wobei die Auswertungseinrichtung (24) ausgelegt ist, um eine Temperaturabhängigkeit der Viskosität des durch die fluidische Reihenschaltung strömenden Fluids bei der Bestimmung der Strömungsrate zu berücksichtigen.

**8.** Vorrichtung nach Anspruch 7, die ferner einen Speicher (33) aufweist, in dem Daten, die die Viskosität eines oder mehrerer Fluide widerspiegeln, abgelegt sind.

**9.** Vorrichtung nach einem der Ansprüche 1 bis 8, bei der die Flusswiderstände (12, 16) und die Messbereiche (14, 18) derart ausgelegt sind, dass ein Druckabfall über jede der Flussrestriktionen (12, 16) im Normalbetrieb zumindest 10-mal größer ist als ein Druckabfall über jeden der Messbereiche (14, 18).

**10.** Vorrichtung nach einem der Ansprüche 1 bis 9, bei der die fluidische Reihenschaltung (10) in einem Fluidikmodul (50) gebildet ist, das austauschbar mit einem oder mehreren Modulen koppelbar ist, die den ersten und zweiten Sensor (20, 22) und die Auswertungseinrichtung (24) aufweisen.

**11.** Vorrichtung nach einem der Ansprüche 1 bis 10, bei der die erste und die zweite Flussrestriktion (12, 16) durch zusammengedrückte Bereiche eines elastischen Schlauchs (130) und die Messbereiche (14, 18) durch nicht zusammengedrückte Bereiche des elastischen Schlauchs (130) implementiert sind.

**12.** Vorrichtung nach Anspruch 10, wobei das Fluidikmodul folgende Merkmale aufweist:

einen Modulkörper (50) mit einer strukturierten Modulkörperoberfläche; und
eine Membran (56), die an den Modulkörper (50) angebracht ist und mit der strukturierten Modulkörperoberfläche einen Fluidbereich definiert, wobei der Fluidbereich eine erste Flussrestriktion (12), eine an die erste Flussrestriktion angrenzende erste Messkammer (14), eine an die erste Messkammer angrenzende zweite Flussrestriktion (16) und eine an die zweite Flussrestriktion angrenzende zweite Messkammer (18) aufweist.

**13.** Vorrichtung nach Anspruch 11 mit einem Detektionsmodul (120), das auf den elastischen Schlauch (130) aufsteckbar ist, wobei das Detektionsmodul folgende Merkmale aufweist:

eine erste Klemmvorrichtung (122) zum Klemmen des Detektionsmoduls (120) an den elastischen Schlauch (130) an einer ersten Position längs des elastischen Schlauchs (130);
eine zweite Klemmvorrichtung (124) zum Klemmen des Detektionsmoduls (120) an den elastischen Schlauch (130) an einer zweiten, von der ersten Position beabstandeten Position längs des elastischen Schlauchs (130), wobei die erste und die zweite Klemmvorrichtung (122, 124) ausgebildet sind, um den elastischen Schlauch (130) jeweils definiert zusammenzudrücken, um die erste und die zweite Flussrestriktion zu definieren;
den ersten Sensor (20), der ausgebildet ist, um ein Maß für den zwischen der ersten und zweiten Flussrestriktion in dem elastischen Schlauch (130) herrschenden Druck zu erfassen; und
den zweiten Sensor (22), der ausgebildet ist, um ein Maß für den in einem Schlauchbereich herrschenden Druck zu messen, der auf einer von der ersten Flussrestriktion abgewandten Seite der zweiten Flussrestriktion angeordnet ist.

**14.** Infusionsgerät mit einer Vorrichtung nach einem der Ansprüche 1 bis 11, einem Fluidikmodul nach Anspruch 12 oder einem Detektionsmodul nach Anspruch 13.

**Claims**

**1.** A device for determining at least one flow parameter, comprising:

a fluidic series connection (10) comprising, in this order, a first flow restriction (12), a first measurement area (14), a second flow restriction (16), and a second measurement area (18), a first sensor (20) for detecting a quantitative first measure of the pressure in the first measurement area (14);

a second sensor for detecting a second quantitative measure of the pressure in the second measurement area (18); and

an evaluation means (24) configured to determine, while using the measures detected by the first and second sensors (20, 22), whether there is an occlusion of the first flow restriction (12), of the second flow restriction (16) or of a fluid area adjoining the second measurement area, **characterized in that** the evaluation means is configured to determine

that there is an occlusion of the first flow restriction (12) when the first measure and the second measure indicate a pressure that has a first, low level,

that there is an occlusion of the second flow restriction (16) when the first measure has a pressure having a second, high level, which is higher than the first, low level, and the second measure indicates a pressure of the low first level,

that there is an occlusion of the fluid area adjoining the second measurement area (18) when the first and second measures indicate a pressure of the second, high level, and

that there is no occlusion when the first measure indicates a third pressure which is between the first and second levels, and when the second measure indicates a pressure of a level which lies in the region of the low first level.

2. The device as claimed in claim 1, wherein the evaluation means (24) is further configured to determine, while using the measures detected by the first and second sensors (20, 22), a flow rate of a fluid flowing through the series connection, wherein the device further comprises pumping means (28) for providing a fluid flow through the fluidic series connection (10), the evaluation means (24) being configured to control the pumping means (28) on the basis of the measures detected so as to obtain a desired flow rate.

3. The device as claimed in claims 1 or 2, wherein the fluid area adjoining the second measurement area is a cannula (30).

4. The device as claimed in any of claims 1 to 3, wherein the measurement areas (14, 18) have elastic wall areas (56a, 56b) whose positions depend on the pressures existing in the measurement areas (14, 18), the sensors (20, 22) being configured to measure the positions of the elastic wall areas (56a, 56b).

5. The device as claimed in any of claims 1 to 4, wherein the fluidic series connection (10) comprises structures within a fluidic module body (50) that are covered by a flexible diaphragm (56) at least in the measurement areas (14, 18).

6. The device as claimed in claim 5, wherein the flexible diaphragm (56) extends across the flow restrictions (12, 16), hold-up elements (58, 60) being provided to prevent the flexible diaphragm from deflecting in the area of the flow restrictions.

7. The device as claimed in any of claims 1 to 6, further comprising a temperature sensor (37), the evaluation means (24) being configured to take into account a temperature dependence of the viscosity of the fluid flowing through the fluidic series connection when determining the flow rate.

8. The device as claimed in claim 7, further comprising a memory (33), wherein data reflecting the viscosity of one or more fluids are stored.

9. The device as claimed in any of claims 1 to 8, wherein the flow resistances (12, 16) and the measurement areas (14, 18) are configured such that a pressure drop across any of the flow restrictions (12, 16) in normal operation is at least 10 times larger than a pressure drop across any of the measurement areas (14, 18).

10. The device as claimed in any of claims 1 to 9, wherein the fluidic series connection (10) is formed within a fluidic module (50) which may be exchangeably coupled to one or more modules that comprise the first and second sensors (20, 22) and the evaluations means (24).

11. The device as claimed in any of claims 1 to 10, wherein the first and second flow restrictions (12, 16) are implemented by compressed areas of an elastic tubing (130), and the measurement areas (14, 18) are implemented by non-compressed areas of the elastic tubing (130).

12. The device as claimed in claim 10, wherein the fluidic module comprises:

a module body (50) having a structured module body surface; and
a diaphragm (56) mounted on the module body (50) and defining a fluid area together with the structured module body surface, the fluid area comprising a first flow restriction (12), a first measurement chamber (14) adjoining the first flow restriction, a second flow restriction (16) adjoining the first measurement chamber, and a second measurement chamber (18) adjoining the second flow restriction.

13. The device as claimed in claim 11, comprising a detection module (120) which may be fitted onto the elastic tubing (130), the detection module (120) comprising:

a first clamping device (122) for clamping the detection module (120) onto the elastic tubing (130) at a first position alongside the elastic tubing (130);

a second clamping device (124) for clamping the detection module (120) onto the elastic tubing (130) at a second position alongside the elastic tubing (130), said second position being spaced apart from the first position,

the first and second clamping devices (122, 124) being configured to compress the elastic tubing (130) in a defined manner in each case so as to define the first and second flow restrictions;

the first sensor (20), which is configured to detect a measure of the pressure existing between the first and second flow restrictions within the elastic tubing (130); and

the second sensor (22), which is configured to measure a measure of the pressure existing within a tubing area arranged on a side of the second flow restriction that faces away from the first flow restriction.

14. An infusion apparatus comprising a device as claimed in any of claims 1 to 11, a fluidic module as claimed in claim 12, or a detection module as claimed in claim 13.

**Revendications**

1. Dispositif de détermination d'au moins un paramètre d'écoulement, avec:

un circuit série fluidique (10) présentant, dans cet ordre, une première restriction d'écoulement (12), une première zone de mesure (14), une deuxième restriction d'écoulement (16) et une deuxième zone de mesure (18),

un premier capteur (20) destiné à détecter une première mesure quantitative de la pression dans la première zone de mesure (14);

un deuxième capteur destiné à détecter une deuxième mesure quantitative de la pression dans la deuxième zone de mesure (18); et

un moyen d'évaluation (24) conçu pour déterminer, à l'aide des mesures détectées par le premier et le deuxième capteur (20, 22), s'il existe une obstruction de la première restriction d'écoulement (12), de la deuxième restriction d'écoulement (16) ou d'une zone de fluide se raccordant à la deuxième zone de mesure, **caractérisé par le fait que** le dispositif d'évaluation est conçu pour déterminer

qu'il existe une obstruction de la première restriction d'écoulement (12) lorsque la première mesure et la deuxième mesure indiquent une pression qui présente un premier niveau faible, qu'il existe une obstruction de la deuxième restriction d'écoulement (16) lorsque la première mesure présente une pression au deuxième niveau élevé qui est supérieur au premier niveau faible et que la deuxième mesure indique une pression du premier niveau faible,

qu'il existe une obstruction de la zone de fluide adjacente à la deuxième zone de mesure (18) lorsque la première et la deuxième mesure indiquent une pression du deuxième niveau élevé, et

qu'il n'existe pas d'obstruction lorsque la première mesure indique une troisième pression située entre le premier et le deuxième niveau et que la deuxième mesure indique une pression à un niveau situé dans la plage du premier niveau faible.

2. Dispositif selon la revendication 1, dans lequel le moyen d'évaluation (24) est par ailleurs conçu pour déterminer, à l'aide des mesures détectées par le premier et le deuxième capteur (20, 22), un débit d'écoulement d'un fluide circulant dans le circuit série, dans lequel le dispositif présente par ailleurs un moyen de pompe (28) destiné à permettre un écoulement de fluide à travers le circuit série fluidique (10), dans lequel le moyen d'évaluation (24) est conçu pour commander le moyen de pompe (28) sur base des mesures détectées, pour obtenir un débit d'écoulement souhaité.

3. Dispositif selon la revendication 1 ou 2, dans lequel la zone de fluide se raccordant à la deuxième zone de mesure est une canule (30).

4. Dispositif selon l'une des revendications 1 à 3, dans lequel les zones de mesure (14, 18) présentent des zones de paroi élastiques (56a, 56b) dont la position dépend des pressions régnant dans les zones de mesure (14, 18), dans lequel les capteurs (20, 22) sont conçus pour mesurer la position des zones de paroi élastiques (56a, 56b).

5. Dispositif selon l'une des revendications 1 à 4, dans lequel le circuit série fluidique (10) présente des structures dans un corps de module fluidique (50) qui sont recouvertes au moins dans les zones de mesure (14, 18) par une membrane souple (56).

6. Dispositif selon la revendication 5, dans lequel la membrane souple (56) s'étend au-dessus des restrictions d'écoulement (12, 16), dans lequel sont prévus des éléments de retenue (58, 60) pour empêcher une déviation de la membrane souple à l'endroit des restrictions d'écoulement.

7. Dispositif selon l'une des revendications 1 à 6, présentant par ailleurs un capteur de température (37), dans lequel le moyen d'évaluation (24) est conçu pour tenir compte d'une dépendance de la température de la viscosité du fluide s'écoulant dans le circuit série fluidique lors de la détermination du débit d'écoulement.

8. Dispositif selon la revendication 7, présentant par ailleurs une mémoire (33) dans laquelle sont mémorisées des données reflétant la viscosité d'un ou plusieurs fluides.

9. Dispositif selon l'une des revendications 1 à 8, dans lequel les résistances à l'écoulement (12, 16) et les zones de mesure (14, 18) sont conçues de sorte qu'une chute de pression à chacune des restrictions d'écoulement (12, 16) soit, en fonctionnement normal, au moins 10 fois supérieure à une chute de pression à chacune des zones de mesure (14, 18).

10. Dispositif selon l'une des revendications 1 à 9, dans lequel le circuit série fluidique (10) est formé dans un module fluidique (50) qui peut être couplé de manière interchangeable à un ou plusieurs modules qui présentent le premier et le deuxième capteur (20, 22) et le moyen d'évaluation (24).

11. Dispositif selon l'une des revendications 1 à 10, dans lequel la première et la deuxième restriction d'écoulement (12, 16) sont mises en oeuvre par des zones comprimées d'un tube élastique (130) et les zones de mesure (14, 18) par des zones non comprimées du tube élastique (130).

12. Dispositif selon la revendication 10, dans lequel le module fluidique présente les caractéristiques suivantes :

un corps de module (50) avec une surface de corps de module structurée ; et
une membrane (56) qui est placée sur le corps de module (50) et qui définit une zone de fluide avec la surface du corps de module structurée, où la zone de fluide présente une première restriction d'écoulement (12), une première chambre de mesure (14) adjacente à la première restriction d'écoulement, une deuxième restriction d'écoulement (16) adjacente à la première chambre de mesure et une deuxième chambre de mesure (18) adjacente à la deuxième restriction d'écoulement.

13. Dispositif selon la revendication 11, avec un module de détection (120) pouvant être enfiché sur le tube élastique (130), dans lequel le module de détection présente les caractéristiques suivantes :

un premier dispositif de serrage (122) destiné à serrer le module de détection (120) sur le tube élastique (130) à une première position le long du tube élastique (130) ;
un deuxième dispositif de serrage (124) destiné à serrer le module de détection (120) sur le tube élastique (130) à une deuxième position, distante de la première position, le long du tube élastique (130),
dans lequel le premier et le deuxième dispositif de serrage (122, 124) sont conçus pour comprimer le tube élastique (130) chaque fois de manière définie, pour définir la première et la deuxième restriction d'écoulement ;
le premier capteur (20) conçu pour détecter une mesure de la pression régnant entre la première et la deuxième restriction d'écoulement dans le tube élastique (130) ; et
le deuxième capteur (22) est conçu pour mesurer une mesure de la pression régnant dans une zone de tuyau flexible qui est disposée d'un côté de la deuxième restriction d'écoulement qui est opposé à la première restriction d'écoulement.

14. Appareil de perfusion avec un dispositif selon l'une des revendications 1 à 11, un module fluidique selon la revendication 12 ou un module de détection selon la revendication 13.

FIG 1

FIG 2

FIG 3A

FIG 3B

FIG 4A

FIG 4B

FIG 4C

FIG 5

**FIG 6**

FIG 7

FIG 8  (Stand der Technik)

FIG 9  (Stand der Technik)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20040127844 A1
- US 20070151346 A1
- US 5720721 A
- US 4994035 A
- EP 1769738 A1
- EP 1818664 A1
- EP 0401524 A2
- WO 9603168 A
- DE 4308313 A1
- EP 1772162 A